# EUROPÄISCHE PATENTSCHRIFT

(11) **EP 1 096 942 B1**
(45) Veröffentlichungstag und Bekanntmachung des Hinweises auf die Patenterteilung: **19.11.2003**
(21) Anmeldenummer: 99927748.6
(22) Anmeldetag: 22.05.1999
(51) Int. Cl.: A61K 35/74, A61P 31/12

(54) **VERWENDUNG EINES ARZNEIMITTELS MIT EINEM GEHALT AN TYROTHRICIN ZUR BEHANDLUNG VON VIRUSINFEKTIONEN**
USE OF A MEDICINE CONTAINING A QUANTITY OF TYROTHRICINE FOR THE TREATMENT OF VIRAL INFECTIONS
UTILISATION D'UN MEDICAMENT PRESENTANT UNE CERTAINE TENEUR EN TYROTHRICINE POUR LE TRAITEMENT D'INFECTIONS VIRALES

(30) Priorität: 26.05.1998 DE 19823318
(43) Veröffentlichungstag der Anmeldung: 09.05.2001
(73) Patentinhaber: Engelhard Arzneimittel GmbH & Co. KG, 61138 Niederdorfelden (DE)
(72) Erfinder: RUNKEL, Frank, D-35418 Buseck (DE)
(74) Vertreter: Weller, Wolfgang, Dr.
(86) Internationale Anmeldenummer: EP9903539
(87) Internationale Veröffentlichungsnummer: WO99061011

(56) Entgegenhaltungen:
- GROSSGEBAUER K ET AL: "[ Antiviral activity of tyrothricin against Sendai virus in suspension tests (author's transl)]. Die antivirale Wirkung von Tyrothricin im Suspensionsversuch am Beispiel des Sendai- Virus." ZENTRALBLATT FUR BAKTERIOLOGIE, PARASITENKUNDE, INFEKTIONSKRANKHEITEN UND HYGIENE. ERSTE ABTEILUNG ORIGINALE. REIHE B: HYGIENE, PRAVENTIVE MEDIZIN, (1978 MAY) 166 (4-5) 434-42. , XP002121348
- BIOULAC P.: "[Do's and dont's in oral aphthae]. QUE FAIRE ET NE PAS FAIRE EN PRESENCE D'APHTES BUCCAUX?." BORDEAUX MEDICAL, (1977) 10/24 (1705-1706). CODEN: BOMEBE, XP002121349
- HARTMANN, D. ET AL: "Protective test with tyrothricin on herpes simplex virus infected mice" ARZNEIM.-FORSCH. (1979), 29(1), 50-4 , XP002121350
- GOTTWALD W.: "[Clinical aspects, differential diagnosis and treatment of herpes zoster]. UBER KLINIK, DIFFERENTIALDIAGNOSE UND THERAPIE DES ZOSTER." THERAPIEWOCHE, (1981) 31/41 (6665-6672). CODEN: THEWA6, XP002121351

## Beschreibung

Die Erfindung betrifft eine Verwendung von Tyrothricin zur Herstellung eines Arzneimittels zur Behandlung von Infektionen mit Viren der Familie der Poxviridae.

Viren sind Krankheitserreger, die sich nicht selbständig vermehren können, sondern zum Zwecke ihrer Vermehrung in lebende Zellen, sogenannte Wirtszellen, eindringen. Dort programmieren sie den zellulären Stoffwechsel derart um, daß eine Vielzahl von Nachkommen-Viren gebildet werden. Diese verlassen die Wirtszelle und befallen umliegende weitere Wirtszellen, wo sich der Vorgang wiederholt, und so fort.

Viren sind sehr einfach aufgebaute Krankheitserreger. Sie enthalten eine Nukleinsäure, die die genetische Information des Virus beinhaltet, und zwar entweder eine einzelsträngige oder eine doppelsträngige DNA oder RNA. Die Nukleinsäure ist von einem Proteinmantel, dem Capsid, umgeben. Der Komplex aus Nukleinsäure und Protein wird als Nukleocapsid bezeichnet. Bei den behüllten Viren ist das Nukleocapsid von einer Lipidmembran eingeschlossen.

Mit einer Länge von 20 bis 300 Nanometern sind Viren wesentlich kleiner als Bakterien und können daher bspw. aus Blut nicht über Bakterienfilter abgetrennt werden.

Die Klassifikation von Viren erfolgt einerseits nach ihrer Nukleinsäure in DNA- und RNA-Viren und andererseits nach dem Vorhandensein einer Lipidhülle in behüllte und unbehüllte Viren.

Eine Virusinfektion verläuft in mehreren definierten Phasen (siehe Pschyrembel, 258. Auflage, S. 1667):
1. Adsorption:
   In der Adsorptionsphase lagert sich das Virus an seine Wirtszelle an. Bei behüllten Viren, die in ihrer Lipidhülle meist viruseigene (Glyco-)Proteine tragen, erfolgt die
   Adsorption über eine spezifische Interaktion der Virushüllproteine mit Membranproteinen der Wirtszelle, den Virusrezeptoren. Diese Interaktion von Virushüllproteinen und zellulären Virusrezeptoren bestimmt das Wirtsspektrum des Virus. Viren können nämlich immer nur ganz bestimmte Zelltypen befallen, die die jeweiligen Virusrezeptoren tragen.
2. Penetration und Uncoating:
   Zur Penetration, dem Eindringen des Virus in die Wirtszelle, verschmilzt die Virushülle mit der Zellmembran (behüllte Viren). Bei unbehüllten Viren wird das Virus auf andere Weise, bspw. durch Pinozytose, ins Zellinnere eingeschleust. Im Zellinneren zerfällt das Nukleocapsid in Nukleinsäure und Proteine, dieser Vorgang wird als Uncoating bezeichnet. Das Uncoating findet entweder im Cytoplasma oder im Zellkern statt.
3. Replikation:
   Bei der Replikation wird die viruseigene Nukleinsäure und, davon ausgehend, die viralen Proteine vielfach multipliziert. Die virale Replikation erfolgt nach den verschiedensten Mechanismen, wobei immer wirtszelleigene Enzyme, Nukleotide, Aminosäuren und Energie verwendet werden.
4. Maturation und Freisetzung:
   Die Nachkommen-Nukleinsäuren verbinden sich mit Virusproteinen zu neuen Nukleocapsiden (Virusreifung, Assembly).
   Dann erfolgt der Austritt der Nukleocapside aus der Wirtszelle nach unterschiedlichen Mechanismen. Behüllte Viren verlassen ihre Wirtszelle meist durch "Budding" (Knospung), wobei die Lipidhülle beim Austritt aus der Zelle aus der Zellmembran erworben wird. Bei diesem Vorgang werden auch die Virushüllproteine in die Lipidhülle eingelagert.

Die Nachkommen-Viren befallen dann Wirtszellen in der Nachbarschaft der ursprünglich befallenen Wirtszelle, wo sie sich wiederum vermehren und von dort aus weitere Zellen befallen.

In der Regel geht die Wirtszelle aufgrund der Virusvermehrung, die die übrigen Synthesevorgänge der Zelle meist vollständig blockiert, zugrunde.

Im Gegensatz zu der Vermehrung von Bakterien, die durch eine Vielzahl bekannter Antibiotika unterbunden werden kann, sind bisher nur wenige Mittel bekannt, die die Vermehrung von Viren verhindern.

Der Grund hierfür besteht darin, daß sich die Viren zu ihrer Vermehrung wirtseigene Mechanismen zunutze machen. Daraus folgt, daß die meisten Virostatika, also chemische Verbindungen, die zur Therapie von Virusinfektionen eingesetzt werden, auch für die Wirtszellen und somit den Wirtsorganismus toxisch sind.

Die wenigen bekannten Virostatika greifen in spezifische Mechanismen ein, die für die jeweiligen Virusfamilien oder sogar nur für einzelne Virustypen charakteristisch sind.

Daher sind die bekannten Virostatika meist nur wirksam gegenüber ganz bestimmten Virusfamilien oder inhibieren nur einzelne Virustypen.

Die bisher bekannten Virostatika beruhen auf drei Wirkprinzipien (siehe Pschyrembel, 258. Auflage, S.1668):
a) Verhinderung der Viruspenetration und/oder des Uncoating (siehe oben Schritt 2). Dazu werden zyklische Amine wie bspw. Amantadin eingesetzt.
b) Verhinderung der Virusreplikation (siehe oben Schritt 3), z.B. durch Nukleosid-Analoga wie Aciclovir oder Zidovudin.
c) Verhinderung der Maturation und Freisetzung (siehe oben Schritt 4), bspw. mit Inhibitoren viruseigener Reifungsproteine. Inhibitoren der HIV-Protease sind hier als Beispiel zu nennen.

Zu den bekanntesten Virostatika zählt das Nucleosid-Analog Aciclovir, das die Vermehrung von Herpes simplex und verwandten Viren spezifisch blockiert (siehe Roche, Lexikon Medizin, Urban & Schwarzenberg, 3. Auflage, S. 13). Zur topischen Anwendung wird es bspw. als "Lippencreme" von einer Vielzahl verschiedener Anbieter verkauft.

Ein weitaus weniger bekanntes antivirales Mittel ist Tyrothricin.

Tyrothricin ist ein Gemisch aus zyklischen und linearen Polypeptiden, das zu 80 % aus dem Antibiotikum Tyrocidin und zu 20 % aus dem Antibiotikum Gramicidin, beide aus Bacillus brevis isoliert, besteht. Tyrothricin hat ein überraschend breites Wirkspektrum. Es hat in erster Linie eine antibakterielle Wirkung gegen verschiedene Bakterien und Kokken und wirkt darüber hinaus gegen einige Hefepilze und Protozoen.

Es ist außerdem bekannt, daß Tyrothricin gegen Influenza-, Mumps- und Herpes simplex-Viren eingesetzt werden kann.

Diese Viren stammen aus unterschiedlichen Virusfamilien. Das Influenza Virus gehört zu den behüllten RNA-Viren, und zwar zu der Familie der Orthomyxoviridae. Das Mumps-Virus ist ebenfalls eine behülltes RNA-Virus und gehört zu der Familie der Paramyxoviridae. Das Herpes simplex-Virus ist dagegen ein behülltes DNA-Virus und gehört zur Familie der Herpesviridae.

Es ist Aufgabe der vorliegenden Erfindung, weitere Verwendungen von Tyrothricin zur Herstellung eines Arzneimittels bereitzustellen.

Diese Aufgabe wird durch die Verwendung von Tyrothricin zur Herstellung eines derartigen Arzneimittels zur Behandlung von Infektionen mit Viren der Familie der Poxviridae gelöst.

Die Familie der Poxviridae oder Pockenviren ist eine heterogene Familie, deren Mitglieder große, behüllte DNA-Viren sind.

Die Pockenviren haben Durchmesser von 230 bis 300 Nanometern. Ihre komplexe Lipidhülle umschließt ein meist quaderförmiges Nukleocapsid.

Die Familie der Poxviridae wird unterteilt in die Parapoxviren und die Orthopoxviren. Der berühmteste Vertreter der Orthopoxviren ist das eigentliche Pockenvirus, das Variola oder Pocken verursacht. Aufgrund einer weltweiten Pockenimpfkampagne der WHO gilt die Variola inzwischen als ausgerottet.

Ein weiteres, bisher noch unklassifiziertes, verwandtes Virus ist das Molluscum contagiosum-Virus, das die sogenannten Dellwarzen verursacht.

In einer weiter unten ausgeführten Studie konnte nun erstmals belegt werden, daß ein Arzneimittel, das einen Gehalt an Tyrothricin aufweist, auch gegen ein Mitglied der Familie der Poxviridae, nämlich gegen das Molluscum contagiosum-Virus, antiviral wirksam ist.

Dieses Ergebnis war vollkommen überraschend, da die Familie der Poxviridae nicht mit den oben genannten Virusfamilien, zu denen das Influenza-Virus, das Mumps-Virus oder das Herpes simplex-Virus gehört, verwandt sind. Da, wie oben erwähnt, Virostatika immer sehr spezifisch für bestimmte Virustypen oder Virusfamilien sind, konnte nicht davon ausgegangen werden, daß der Wirkstoff Tyrothricin auch gegen Vertreter anderer Virusfamilien eingesetzt werden könnte.

Die erfindungsgemäße Anwendung dieses bekannten Wirkstoffs erweitert das Wirkungsspektrum dieses Wirkstoffs somit erheblich.

Somit ist die der Erfindung zugrundeliegende Aufgabe vollkommen gelöst.

In die Familie der Poxviridae fällt auch das sehr verbreitete Molluscum contagiosum-Virus.

In einer vorteilhaften Weiterbildung der Erfindung wird Tyrothricin zur Herstellung eines Arzneimittels zur Behandlung von Infektionen mit dem Molluscum contagiosum-Virus, insbesondere zur Behandlung der durch dieses Virus verursachten Dellwarzen, die auch Molluscum contagiosum genannt werden, verwendet.

Dieses Virus ist weltweit verbreitet und vermehrt sich im Bereich von Hautzellen. Molluscum contagiosum-Infektionen treten häufig bei Kindern auf und führen gelegentlich zu Epidemien.

Eine Infektion mit dem Molluscum contagiosum-Virus äußert sich in derben, bis zu erbsgroßen Papeln mit zentraler Eindellung, den Dellwarzen. Dellwarzen treten vor allem im Bereich des Gesichts und der Augenlider, am Hals, an den Achseln, sowie im seitlichen Thorax- und Genitalbereich auf.

Sehr häufig sind Dellwarzen auch mit der Hautkrankheit Neurodermitis assoziiert.

Bisher konnte das Krankheitsbild Molluscum contagiosum noch nicht behandelt werden, sondern die Patienten mußten abwarten, bis das Virus "von selbst" wieder verschwindet, d.h. durch das körpereigene Immunsystem eliminiert wird.

Solange das Immunsystem es noch nicht geschafft hat, das Virus zu beseitigen, kann es sich ungehindert ausbreiten, wodurch eine Vielzahl von Dellwarzen entstehen.

In der erwähnten nachstehend vorgestellten Studie an Patienten konnte nun überraschenderweise festgestellt werden, daß das Arzneimittel mit einem Gehalt an Tyrothricin das Abheilen der Dellwarzen herbeiführen kann und somit zur Behandlung von Infektionen mit Molluscum contagiosum-Viren und damit zur Behandlung von Dellwarzen einsetzbar ist.

Beim Aufplatzen von Dellwarzen werden Viren über einen großen Bereich der Umgebung der Dellwarze ausgestreut und können von dort aus neue Hautbereiche befallen und eine Vielzahl neuer Dellwarzen bilden.

Durch großflächiges Auftragen des erfindungsgemäßen Arzneimittels um den Bereich der Dellwarze herum kann die gesamte Umgebung vor einem Neubefall mit Viren geschützt werden. Dies ist auch dann möglich, wenn die Virusinfektion bereits fortgeschritten ist.

Auf diese Weise kann einer Neuinfektion über weite Bereiche der Haut vorgebeugt werden, so daß eine Neuentstehung von Dellwarzen verhindert wird.

Da der Wirkstoff Tyrothricin bereits seit längerer Zeit als Antibiotikum eingesetzt wird, ist bekannt, daß dieser Stoff nicht mit schwerwiegenden Nebenwirkungen verbunden ist.

Eine Ausbreitung des Molluscum contagiosum-Virus und der damit verbundenen Entstehung von Dellwarzen kann somit unter größtmöglicher Vermeidung von Nebenwirkungen bekämpft werden.

Das erfindungsgemäße Arzneimittel ist daher auch zur Prophylaxe gegen die Entstehung von Dellwarzen geeignet. Dies ist z.B. bei den stark gefährdeten Neurodermitikern angebracht.

In einer vorteilhaften Weiterbildung wird ein Arzneimittel verwendet, das einen zusätzlichen Gehalt an einem antiseptischen Mittel aufweist.

Ein antiseptisches Mittel ist ein Mittel gegen Wundinfektion mit möglichst rascher Wirkung, breitem Wirkungsspektrum, geringer systemischer Toxizität und guter Gewebeverträglichkeit zur Erzielung einer Keimfreiheit z.B. an Schleimhäuten und Haut.

Die Verwendung eines antiseptischen Mittels in Verbindung mit einem Arzneimittel, das einen Gehalt an Tyrothricin aufweist, hat den Vorteil, daß eine Vermehrung von weiteren Erregern wie Bakterien und Pilzen in dem Bereich, wo das Arzneimittel aufgetragen wird, zusätzlich vermieden wird.

Da die mit dem Virus infizierten Hautbereiche geschädigt sind, können derartige Sekundärinfektionen leichter auftreten als an unbeschädigter, gesunder Haut. Es ist daher vorteilhaft, Sekundärinfektionen der mit Molluscum contagiosum-Virus infizierten Hautbereiche mit Bakterien oder Pilzen durch ein antiseptisch wirksames Mittel zusätzlich zu bekämpfen. Außerdem verlängert der Zusatz eines antiseptischen Mittels auch die Haltbarkeit des Arzneimittels als solches.

In einer vorteilhaften Ausgestaltung ist das antiseptische Mittel Cetylpyridiniumchlorid.

Cetlypyridiniumchlorid ist eine quarternäre Ammoniumbase, die antiseptisch wirkt und zusätzlich die Oberflächenspannung vermindert und daher den Kontakt der Wirkstoffe des erfindungsgemäßen Arzneimittels mit den zu behandelnden Oberflächen verbessert. Besonders vorteilhaft ist dies, wenn das Arzneimittel im Bereich von Schleimhäuten eingesetzt wird. Durch den Zusatz von Cetylpyridiniumchlorid kann die antivirale Wirksamkeit des erfindungsgemäßen Arzneimittels somit weiter gestärkt werden.

In vorteilhaften Weiterbildungen der Erfindung wird das Arzneimittel mit einem Gehalt an Tyrothricin verwendet, der im Bereich von 0,005 bis 0,5 Gew.-%, vorzugsweise im Bereich von 0,01 bis 0,1 Gew.-% liegt. Höchst bevorzugt ist die Verwendung von Tyrothricin in Mengen von 0,05 Gew.-%.

In der bereits erwähnten Patientenstudie konnte nämlich belegt werden, daß in diesem Bereich eine gute Wirksamkeit des Wirkstoffs Tyrothricin zur therapeutischen Behandlung von Dellwarzen erzielt werden konnte, ohne daß es dabei zu Nebenwirkungen gekommen wäre.

In einer weiteren vorteilhaften Ausgestaltung wird das Arzneimittel in einer topisch anwendbaren Formulierung verwendet.

Durch die topische Anwendung des erfindungsgemäßen Arzneimittels, also die lokale Anwendung des Mittels direkt an Ort und Stelle der Infektion und damit der aktiven Virusvermehrung und - ausbreitung, können die Nebenwirkungen vorteilhafterweise auf ein Minimum reduziert werden.

Es ist weiter von Vorteil, daß das Tyrothricin durch die topische Anwendung direkt mit den infizierten Körperbereichen in Kontakt treten und somit unmittelbar und in hoher Konzentration wirksam werden kann.

In einer weiteren vorteilhaften Ausgestaltung ist das erfindungsgemäße Arzneimittel als Salbe, als Gel, als Puder, als Spray oder als Tinktur formuliert.

Dazu weist das erfindungsgemäße Arzneimittel die bei diesen Formulierungen üblichen Hilfsstoffe auf.

Hierbei ist von Vorteil, daß diese Formulierungen zur topischen, also lokalen Anwendung von Wirkstoffen sehr gut geeignet sind und sich leicht von dem Patienten selbst anwenden lassen.

Als Salbe, Gel oder Puder ist das erfindungsgemäße Arzneimittel vor allem im Hautbereich einsetzbar. Als Spray oder Tinktur kann es vorteilhaft im Bereich von Schleimhäuten, z.B. in der Mundhöhle oder im Genitalbereich eingesetzt werden.

Es versteht sich, daß die vorstehend genannten und die nachstehend noch zu erläuternden Merkmale nicht nur in den jeweils angegebenen Kombinationen, sondern auch in anderen Kombinationen oder in Alleinstellung verwendbar sind, ohne den Rahmen der vorliegenden Erfindung zu verlassen.

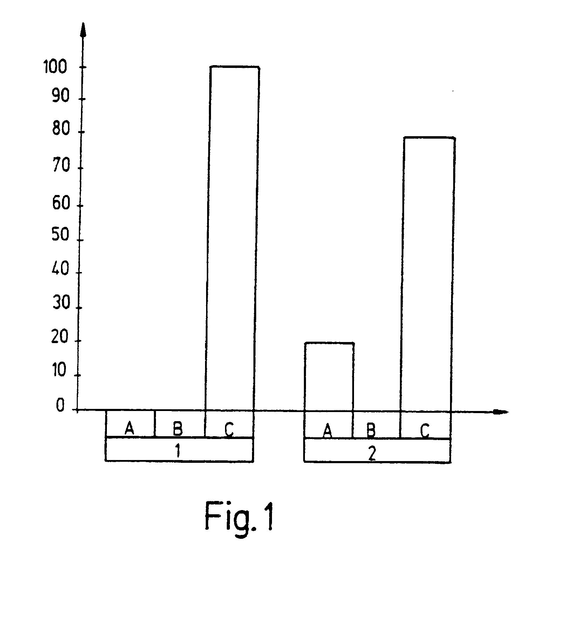

Die Erfindung wird nun unter Bezugnahme auf das nachstehende Beispiel und im Zusammenhang mit der anliegenden Zeichnung näher erläutert, in der
- Fig. 1: ein Balkendiagramm zeigt, das das Ergebnis einer Studie über die Wirkung eines Tyrothricin enthaltenden Arzneimittels bei Molluscum contagiosum (Beispiel 1) wiedergibt.

### Beispiel 1 Untersuchung der Wirkung von Tyrothricin und Dexpanthenol bei Molluscum contagiosum

In dieser Studie wurde die Wirksamkeit eines Arzneimittels mit einem Gehalt an Tyrothricin im Vergleich zu einem Arzneimittel mit einem Gehalt an einem weiteren Wirkstoff, nämlich Dexpanthenol, gegenüber Molluscum contagiosum untersucht.

### Durchführung:

Die Patienten waren Kinder und Jugendliche im Alter von 7 bis 14 Jahren männlichen und weiblichen Geschlechts.

Das Patientenkollektiv umfaßte 20 Patienten.

Davon wurden 10 Patienten mit einer Tyrothricin-Salbe behandelt, die 0,5 mg Tyrothricin und 0,25 mg Cetylpyridiniumchlorid enthielt. Weitere 10 Patienten wurden mit einer Dexpanthenol-Salbe (25 mg Dexpanthenol, Bepanthen-Salbe der Firma Hoffmann-LaRoche) behandelt.

Die Applikation der Salbe erfolgte im Durchschnitt zweimal täglich, die Medikation dauerte durchschnittlich 7 Tage.

Bei der Patientenauswahl wurde darauf geachtet, daß diese erstens keiner Behandlung mit weiteren Virostatika unterworfen waren, daß ihnen zweitens keine Corticosteroide verabreicht wurden und daß sie drittens nicht mit Immunsuppressiva therapiert wurden.

Zur ärztlichen Beurteilung der Therapie wurden folgenden Faktoren bewertet:
1. Abschwellen der Dellwarzen
2. Größe von Juckreiz und Schmerzen
3. Austrocknungseffekt
4. Gewebsgranulation

Anhand dieser Kriterien wurde beurteilt, ob mit den verschiedenen eingesetzten Wirkstoffen eine Abheilung, eine deutliche Besserung oder nur eine geringe bis ungenügende Besserung erreicht wurde.

Darüber hinaus wurde die Lokalisation der Dellwarzen am Patientenkörper festgehalten, wie in der nachstehenden Tabelle 1 gezeigt ist.

**Tabelle 1:**

| Lokalisation der Dellwarzen in Abhängigkeit des eingesetzten Arzneimittels | | | | | |
|---|---|---|---|---|---|
| Wirkstoff | Abdomen | Genital- und Anal- Bereich | Hals und Arme | Gesicht | Gesamtanzahl |
| Dexpanthenol | 3 | 0 | 3 | 4 | 10 |
| Tyrothricin | 2 | 2 | 1 | 5 | 10 |

### Ergebnis:

Die Ergebnisse sind in der nachstehenden Tabelle 2 aufgeführt.

**Tabelle 2:**

| Therapeutische Wirksamkeit von Tyrothricin im Vergleich zu Dexpanthenol bei Molluscum contagiosum | | | | | | | | |
|---|---|---|---|---|---|---|---|---|
| Wirkstoff | Anzahl der Patienten | Anteil der Patienten in % | Erfolg | | | | | |
| | | | Abheilung | Abheilung in % | Deutliche Bessserung | Deutliche Besserung in % | Geringe bis ungenügende Besserung | Geringe bis ungenügende Besserung in % |
| Dexpanthenol | 10 | 100 | 0 | 0 | 0 | 0 | 10 | 100 |
| Tyrothricin | 10 | 100 | 2 | 20 | 0 | 0 | 8 | 80 |

Das in Tabelle 2 aufgeführte Ergebnis ist in Fig. 1 graphisch dargestellt.

Die unter 1 darstellten Säulen repräsentieren die Behandlungserfolge mit Dexpanthenol und unter 2 sind die Ergebnisse mit Tyrothricin alleine aufgeführt.

Mit A ist die vollständige Abheilung der Dellwarzen bei den Patienten bezeichnet, B bezeichnet eine deutliche Besserung und C einen geringen bis ungenügenden Therapieerfolg.

Wie aus Tabelle 2 und Fig. 1 ersichtlich ist, zeigt das Arzneimittel mit einem Gehalt an Tyrothricin in 20 % der Fälle eine vollständige Abheilung der Dellwarzen.

Dieses Ergebnis war vollkommen überraschend, da eine Wirksamkeit von Tyrothricin gegenüber Molluscum contagiosum-Virus bisher noch nicht bekannt war.

Derzeit werden weitere Studien durchgeführt, anhand derer die Wirksamkeit von Tyrothricin zur Behandlung von Molluscum contagiosum näher untersucht wird.

Im Vergleich dazu war bei Anwendung der Dexpanthenol-Salbe alleine in 100 % der Fälle nur ein geringer bis ungenügender Therapieerfolg nachweisbar.

## Patentansprüche

1. Verwendung von Tyrothricin zur Herstellung eines Arzneimittels zur Behandlung von Infektionen mit Viren der Familie der Poxviridae.

2. Verwendung nach Anspruch 1 zur Behandlung von Infektionen mit dem Molluscum contagiosum-Virus.

3. Verwendung nach Anspruch 2 zur Behandlung von Dellwarzen (Molluscum contagiosum).

4. Verwendung nach einem der Ansprüche 1 bis 3, **dadurch gekennzeichnet, daß** das Arzneimittel einen zusätzlichen Gehalt an einem antiseptischen Mittel aufweist.

5. Verwendung nach Anspruch 4, **dadurch gekennzeichnet, daß** daß das antiseptische Mittel Cetylpyridiniumchlorid ist.

6. Verwendung nach einem der Ansprüche 1 bis 5, **dadurch gekennzeichnet, daß** das Arzneimittel einen Gehalt an Tyrothricin im Bereich von 0,005 bis 0,5 Gew.-%, vorzugsweise im Bereich von 0,01 bis 0,1 Gew.-% aufweist.

7. Verwendung nach Anspruch 6, **dadurch gekennzeichnet, daß** der Gehalt an Tyrothricin 0,05 Gew.-% beträgt.

8. Verwendung nach einem der Ansprüche 1 bis 7, **dadurch gekennzeichnet, daß** das Arzneimittel als topisch anwendbares Arzneimittel formuliert ist.

9. Verwendung nach Anspruch 8, **dadurch gekennzeichnet, daß** das Arzneimittel als Salbe, Gel, Puder, Spray oder Tinktur formuliert ist.

## Claims

1. Use of tyrothricin for producing a medicament for the treatment of infections with viruses of the poxviridae family.

2. Use according to Claim 1 for the treatment of infections with the molluscum contagiosum virus.

3. Use according to Claim 2 for the treatment of molluscum contagiosum.

4. Use according to any of Claims 1 to 3, **characterized in that** the medicament has an additional content of an antiseptic agent.

5. Use according to Claim 4, **characterized in that** the antiseptic agent is cetylpyridinium chloride.

6. Use according to any of Claims 1 to 5, **characterized in that** the medicament has a tyrothricin content in the range from 0.005 to 0.5% by weight, preferably in the range from 0.01 to 0.1% by weight.

7. Use according to Claim 6, **characterized in that** the tyrothricin content is 0.05% by weight.

8. Use according to any of Claims 1 to 7, **characterized in that** the medicament is formulated as medicament which can be used topically.

9. Use according to Claim 8, **characterized in that** the medicament is formulated as ointment, gel, dusting powder, spray or tincture.

## Revendications

1. Utilisation de tyrothricine pour la fabrication d'un médicament pour le traitement d'infections dues à des virus de la famille des poxvirus.

2. Utilisation selon la revendication 1 pour le traitement d'infections avec le virus Molluscum contagiosum.

3. Utilisation selon la revendication 2 pour le traitement de l'acné varioliforme (Molluscum contagiosum).

4. Utilisation selon l'une des revendications 1 à 2, **caractérisée en ce que** le médicament contient, en outre une certaine quantité d'un produit antiseptique.

5. Utilisation selon la revendication 4, **caractérisée en ce que** le produit antiseptique est du chlorure de cétylpyridinium.

6. Utilisation selon l'une des revendications 1 à 5, **caractérisée en ce que** le médicament contient une part de tyrothricine de l'ordre de 0,005 à 0,5 % en poids, de préférence de l'ordre de 0,01 à 0,1 % en poids.

7. Utilisation selon la revendication 6, **caractérisée en ce que** la part de tyrothricine est de 0,05 % en poids.

8. Utilisation selon l'une des revendications 1 à 7, **caractérisée en ce que** le médicament est formulé comme un médicament utilisable de manière topique.

9. Utilisation selon la revendication 8, **caractérisée en ce que** le médicament est formulé comme une pommade, un gel, une poudre, un spray ou une teinture.
